# EUROPÄISCHE PATENTANMELDUNG

(11) **EP 1 818 819 A1**
(43) Veröffentlichungstag der Anmeldung: **15.08.2007**
(21) Anmeldenummer: 06100419.8
(22) Anmeldetag: 17.01.2006
(51) Int. Cl.: G06F 9/445, G06F 19/00

(54) **Verfahren zum Organisieren der Software eines Fluidmanagementsystems**

(71) Anmelder: B. Braun Medizintechnologie GmbH, 34212 Melsungen (DE)
(72) Erfinder: Harkányi, Gábor, 1148 Budapest (HU); Osztódi, Tibor, 1031 Budapest (HU); Niemetz, Günter, 34212 Melsungen (DE)
(74) Vertreter: Selting, Günther

(57) **Zusammenfassung**

Das Prozessorsystem (10) einer Patientenbehandlungsmaschine ist mit einer Schnittstelle (32) zum Anschluss eines externen Datenspeichergerätes (40) versehen. Die Software des Prozessorsystems (10) ist unterteilt in eine die Patientenbehandlung steuernde und überwachende Behandlungssoftware (22) und eine die Serviceaktivitäten und/oder Produktionsaktivitäten steuernde und überwachende Tool-Software (26). In dem Prozessorsystem ist die Behandlungssoftware als residente Software und die Tool-Software als nichtresidente Software gespeichert. Über ein externes Datenspeichergerät (40) kann die Tool-Software geladen werden. Außerdem können über das externe Datenspeichergerät Updates durchgeführt werden.

## Beschreibung

Die Erfindung betrifft ein Verfahren zum Organisieren der Software eines Fluidmanagementsystems, welches mindestens ein Prozessorsystem mit CPU, Speicher und Software enthält.

Unter einem Fluidmanagementsystem ist insbesondere ein medizinisches Behandlungsgerät zu verstehen, welches eine extrakorporale Blutbehandlung durchführt, wie beispielsweise eine Hämodialyse oder Hämofiltration. Hierbei wird ein Körperfluid des Patienten, z. B. Blut, einer extrakorporalen Behandlung unterzogen, beispielsweise mit einer Dialysierflüssigkeit. Derartige Fluidmanagementsysteme unterliegen hohen Anforderungen an Zuverlässigkeit und Genauigkeit. Die Nicht-Einhaltung dieser Anforderungen kann zu einer erheblichen Gefährdung für den Patienten führen. Daher ist die Software, die in das Prozessorsystem geladen wird, sehr komplex. Sie umfasst grob unterteilt eine Behandlungssoftware und eine Tool-Software. Die Behandlungssoftware umfasst die Behandlungsprogramme, denen der Patient ausgesetzt wird, und die Tool-Software umfasst Service- und Produktionprogramme. Die Behandlungssoftware muss gelegentlich einem Update unterzogen werden, wozu ein Teil oder die Gesamtheit der Behandlungssoftware aus dem Gerät entfernt und durch eine andere Behandlungssoftware ersetzt wird.

EP 1 227 854 B1 beschreibt ein Software-Update für ein medizinisches Fluidmanagementgerät, bei dem in einem Softwareaktualisierungsmodus unter Berücksichtigung der vorhandenen Versionen der Softwareprogramme und/oder der vorhandenen Prozessorsysteme ermittelt wird, welche Softwareprogramme über die Dateneingabevorrichtung für welche Prozessorsysteme zu laden sind, bevor das Laden der Softwareprogramme in die betreffenden Prozessorsysteme initiiert wird.

Ferner beschreibt DE 44 08 544 C2 ein Verfahren zur Installation von Software-Komponenten auf als Zielrechner fungierenden Datenverarbeitungseinrichtungen. Hierbei überträgt ein auf dem Zielrechner residentes oder vorab von einer Versorgungsanlage übermitteltes Hilfsprogramm ein für die nachfolgenden Schritte vorbereitetes Betriebssystem zum Zielrechner, wo das Hilfsprogramm installiert und gestartet wird.

In EP 0 457 940 A1 ist das Herunterladen von Software in einen medizinischen Monitor mit Hilfe einer Zusatzbox beschrieben.

Bei einem medizinischen Fluidmanagementsystem existiert ein hoher Aufwand an Speicherplatz. Ein Teil des Speicherplatzes wird für Behandlungssoftware benutzt, die beispielsweise eine Dialysebehandlung betrifft. Die Behandlung muss nicht nur gesteuert und überwacht, sondern auch ausführlich dokumentiert werden. Ferner ist gelegentlich eine Freigabeprozedur erforderlich, bei der mehrere Personen ihre Identifikationskennzeichen in den Computer eingeben, um bestimmte Behandlungseinstellungen an der Maschine vornehmen zu können.

Ein anderer Teil der Gerätesoftware kann als Tool-Software bezeichnet werden. Dieser umfasst die Serviceaktivitäten und Produktionsaktivitäten. Eine Notwendigkeit für ein Update der Gerätesoftware wird in den meisten Fällen durch die Tool-Software ausgelöst. Die Behandlungssoftware ist dagegen viel seltener betroffen.

Der Erfindung liegt die Aufgabe zugrunde, ein Verfahren anzugeben, mit dem die Verwaltung und das Updaten der Software eines Prozessorsystems vereinfacht wird.

Das erfindungsgemäße Verfahren ist durch die Merkmale des Patentanspruchs 1 definiert. Hiernach ist vorgesehen, dass die Software unterteilt ist in eine die Patientenbehandlung steuernde und überwachende Behandlungssoftware und eine die Serviceaktivitäten und Produktionsaktivitäten steuernde und überwachende Tool-Software, und dass in dem Prozessorsystem die Behandlungssoftware als residente Software und die Tool-Software als nicht-residente Software gespeichert werden.

Die Erfindung geht von dem Gedanken aus, dass bei einem medizinischen Fluidmanagementsystem etwa 20% der Speicherkapazität für die Behandlungssoftware benötigt werden und etwa 80% für die Tool-Software. Die Tool-Software benötigt Updates viel häufiger als die Behandlungssoftware. Daher wird in das Prozessorsystem nur die Behandlungssoftware als residente Software übernommen, während die Tool-Software außerhalb des Prozessorsystems abgespeichert wird.

Unter residenter Software ist eine Software zu verstehen, die dauerhaft installiert wird, während nicht-residente Software beim Abschalten der Stromversorgung bzw. beim Ausschalten des Gerätes gelöscht wird. Das Prozessorsystem enthält während der Patientenbehandlung nur die Behandlungssoftware. Bei der Herstellung der Maschine und bei der Wartung wird dagegen die Tool-Software benötigt. Bei der Herstellung erfolgt ein schrittweises Hochfahren der Maschine bei einem sogenannten Run-In-Stress, bei dem insbesondere die Pumpen geprüft werden. Hierbei erfolgt zum Beispiel auch eine Überwachung von Temperaturen und Drücken und es werden Kalibrierdaten gewonnen. Die Serviceaktivitäten umfassen die Prüfung und Wartung der Maschinen, die Fehlerdiagnose sowie die Feststellung und Herausziehung von Trends. Der Wartungstechniker, der die Tool-Software installiert, verfügt über das externe Datenspeichergerät, um die Tool-Software auf das Prozessorsystem zu übertragen. Dadurch kommt das Prozessorsystem mit einem Speicher von geringerer Kapazität aus, weil die Tool-Software nicht permanent gespeichert bleiben muss.

Die Erfindung ermöglicht es, die Behandlungssoftware für den Vorgang der Patientenbehandlung ausführlich zu dokumentieren, während die Tool-Software von der Dokumentation ausgenommen wird. Daher wird insgesamt der Dokumentationsaufwand verringert und auch die Freigabeprozeduren, die nur die Behandlungssoftware betreffen, werden verringert. Die Erfindung erleichtert insbesondere auch das Update einer Software. Da die Software in mindestens zwei Komponenten, nämlich Behandlungssoftware und Tool-Software, unterteilt ist, braucht nur die Behandlungssoftware durch das Update erfasst zu werden. Es ist sogar möglich, ein Update der Tool-Software außerhalb des Prozessorsystems durchzuführen, beispielsweise in dem externen Datenspeichergerät oder in einem separaten PC, in dem die Tool-Software einschließlich der Ein- und Ausgabedatei aufbewahrt wird. Während dieses Updates wird das Prozessorsystem der Maschine nicht blockiert, so dass es für die Patientenbehandlung benutzt werden kann.

Auch die residente Software, die im Prozessorsystem gespeichert ist, kann einem Update unterzogen werden. Dieses Update wird vorzugsweise mit dem externen Datenspeichergerät durchgeführt. Zu diesem Zweck ist das Prozessorsystem imstande, Angaben über seine gespeicherte Software und Daten an das externe Datenspeichergerät zu übertragen. Das externe Datenspeichergerät kann auch zum Abfragen von Behandlungsinformationen aus dem Prozessorsystem benutzt werden. Die Ein-/Ausgabe des externen Datenspeichergerätes wird entweder durch eine residente oder eine nicht-residente Ausführungssoftware des Prozessorsystems gesteuert. Daher braucht das externe Datenspeichergerät nicht notwendigerweise einen eigenen Prozessor. Es reicht generell eine Speicherfunktion aus. Alternativ besteht jedoch die Möglichkeit, das externe Datenspeichergerät mit einem eigenen Prozessor auszustatten, und dann auch die Ein-/Ausgabe mit dem eigenen Prozessorsystem zu steuern.

In dem Prozessorsystem wird die Behandlungssoftware als residente Software gespeichert und ausgeführt, während die Tool-Software als nicht-residente Software geladen, gespeichert und ausgeführt wird.

Die Tool-Software ist unterteilbar in Funktionen, wie Software-Update, Serviceunterstützung, Produktionsunterstützung, so dass der Speicherplatz in dem Prozessorsystem nur die Größe einer Teilfunktion besitzen muss, die jeweils für den aktuellen Betrieb benötigt wird.

Die Erfindung betrifft ferner eine Patientenbehandlungsmaschine mit mindestens einem Prozessorsystem, das eine CPU sowie Speicher und darin gespeicherte Software enthält. Eine derartige Patientenbehandlungsmaschine ist durch den Patentanspruch 11 definiert.

Die abhängigen Ansprüche beziehen sich jeweils auf zweckmäßige Ausführungsformen bzw. Weiterbildungen der Gegenstände der unabhängigen Ansprüche 1 und 11.

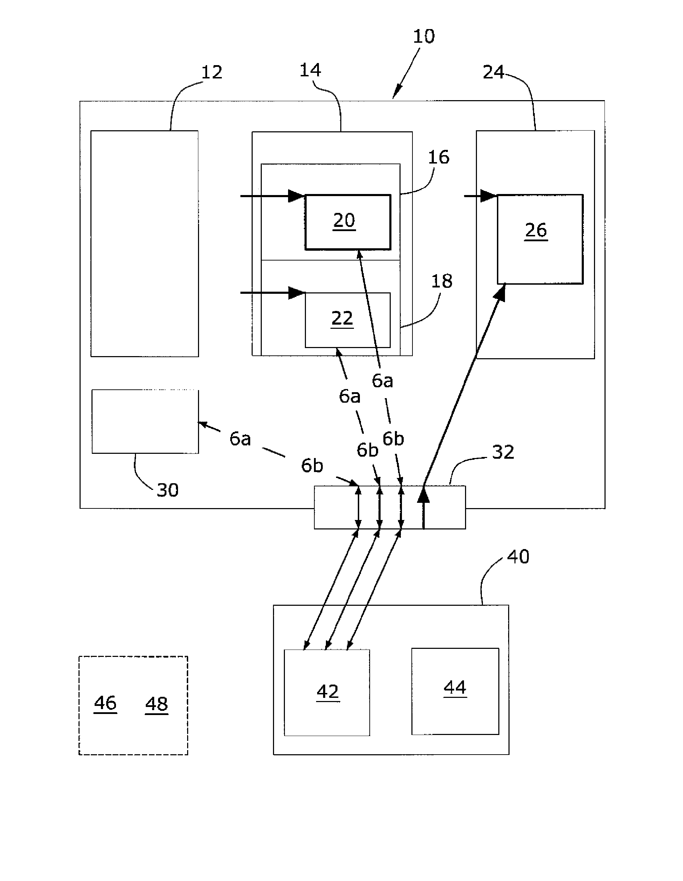

Im Folgenden wird unter Bezugnahme auf die einzige Figur der Zeichnung ein Ausführungsbeispiel der Erfindung näher erläutert.

Die Zeichnung zeigt ein Blockschema eines Prozessorsystems nach der vorliegenden Erfindung.

Das Prozessorsystem 10 ist ein Computer, der Teile der Steuer- und Überwachungseinheit einer (nicht dargestellten) Fluidbehandlungsmaschine zur extrakorporalen Blutbehandlung vom Patienten darstellt. Bei der Blutbehandlungsmaschine handelt es sich beispielsweise um eine Dialysemaschine oder eine Maschine für die Blutplasmabehandlung.

Das Prozessorsystem 10 enthält einen Mikrocontroller mit CPU 12 und einem Arbeitsspeicher. Ferner enthält das Prozessorsystem einen ersten Speicher 14 für residente Programme und einen zweiten Speicher 24 für nicht-residente Programme. Der erste Speicher 14 enthält einen ersten Speicherbereich 16 und einen zweiten Speicherbereich 18. Der erste Speicherbereich 16 ist ein nichtflüchtiger Speicher zur Speicherung einer fest integrierten BIOS-Software (Basic Input/Output System) 20.

Der zweite Speicherbereich 18 des ersten Speichers 14 enthält ein residentes Ausführungsprogramm (executable program) 22.

Der zweite Speicher 24 ist ein RAM zur Speicherung nicht-residenter Programme und Daten. Er enthält unter anderem die nicht-residente Tool-Software 26 und Daten.

Zu dem Prozessorsystem 10 gehören ferner Peripheriegeräte 30 wie Festplatte, Tastatur, Bildschirm, Drucker und Ähnliches.

Die Speicher 14 und 24 sowie die Peripheriegeräte korrespondieren mit der CPU 12 sowie mit einer Schnittstelle 32 jeweils bidirektional. An die Schnittstelle 32 kann über ein Steckersystem ein externes Datenspeichergerät 40 angeschlossen werden. Dieses enthält sowohl Eingabe/Ausgabe-Dateien 42 der Behandlungssoftware und der Toolsoftware sowie Ausführungscode-Dateien für die Behandlungssoftware und BIOS, als auch Ausführungscode-Dateien 44 für die Tool-Software.

In einem Speicher, der im externen Datenspeichergerät 40 separat vorgesehen ist, ist die Tool-Software gespeichert, nämlich eine Software 46 für die Serviceaktivitäten und eine Software 48 für die Produktionsaktivitäten. Beide Softwares können von dem externen Datenspeichergerät 40 an das Prozessorsystem 10 geliefert werden. Das Softwareprogramm 20 zum Laden und Starten der nicht-residenten Tool-Software 26 ist ein BIOS, das mit Abschaltung des Prozessorsystems nicht gelöscht wird. Das Programm wird unter Steuerung der CPU 12 ausgeführt. Das Softwareprogramm 22 zur Ausführung der residenten Behandlungssoftware wird von dem BIOS 20 gestartet und ist ein Softwareprogramm, das unter Steuerung der CPU 12 ausgeführt und bei Abschaltung des Prozessorsystems erhalten bleibt.

Die Softwareprogramme, die Serviceaktivitäten oder Produktionsaktivitäten betreffen, gehören zu den nicht-residenten Softwareprogrammen 26. Diese Softwareprogramme werden von dem BIOS 20 geladen und gestartet. Zu den Serviceprogrammen gehört auch das Software-Updating der residenten Programme 20 und 22.

Von den Doppelpfeilen 6a und 6b gibt jeweils die Pfeilrichtung 6b den Datentransfer an das externe Datenspeichergerät 40 an und die Pfeilrichtung 6a gibt den Ladevorgang eines Softwareprogramms und den Datentransfer vom externen Datenspeichergerät 40 in den entsprechenden internen Speicher an.

Das BIOS 20 bildet den integrierten Softwareprogrammteil des Prozessorsystems von dessen Herstellung an. An ihm kann durch das externe Datenspeichergerät ein Upgrade ausgeführt werden. Die BIOS-Software 20 wird durch ein von einer Hardware erzeugtes Signal nach dem Einschalten des Gerätes gestartet und lädt und startet ihrerseits entweder die nicht-residente Software 26 oder startet die residente Software 22. Die residente Software 22 und die nicht-residente Software können über die Schnittstelle 32 von dem externen Speichergerät Daten und Programme in den Speicher 24 laden oder Daten in den Speicher 40 speichern.

Das Update der Behandlungssoftware geschieht in folgenden Schritten:
- BIOS 20 lädt ein spezifisches Programm für die Kommunikation mit dem externen Datenspeichergerät 40 von dem externen Datenspeichergerät 40 in den Speicher 24 und startet dieses Programm zur Durchführung des Ladens der Ausführungscode-Datei 44 aus dem externen Datenspeichergerät 40 und zur Speicherung in den nicht-residenten Speicher 24 als Update-Programm 26.
- BIOS 20 startet das Update-Programm 26.
- Das Update-Programm 26 führt den Austausch der Behandlungssoftware 22 aus, indem es die Datei oder Dateien aus dem Speicher für die Eingabe-Dateien 42 in den residenten Speicher 18 für die Behandlungssoftware 22 speichert.

Das externe Datenspeichergerät ist vorzugsweise ein Speicherstick oder Flashdrive, der an den USB-Anschluss des Prozessorsystems 10 anschließbar ist.

Andere Möglichkeiten für das externe Datenspeichergerät 40 sind Disketten, CD, DVD, HDD (Hard-Disk-Drive) oder externe Computer, wie beispielsweise ein Laptop. Außerdem können die Softwares 46 und 48 auch über ein Datennetz, z. B. ein LAN in Form eines Ethernet, dem externen Datenspeichergerät 40 oder direkt der Schnittstelle 32 zugeführt werden.

## Patentansprüche

1. Verfahren zum Organisieren der Software eines Fluidmanagementsystems, das mindestens ein Prozessorsystem (10) mit CPU (12), Speichereinrichtung und darin gespeicherter Software enthält,
**dadurch gekennzeichnet,**
**dass** die Software unterteilt ist in eine Patienten behandlung steuernde und überwachende Behandlungssoftware (22) und eine die Serviceaktivitäten und/oder Produktionsaktivitäten steuernde und überwachende Tool-Software (26) und
**dass** in dem Prozessorsystem (10) die Behandlungssoftware als residente Software (22) und die Tool-Software als nicht-residente Software (26) gespeichert werden.

2. Verfahren nach Anspruch 1, **dadurch gekennzeichnet, dass** zu der nicht-residenten Software auch eine Software zur Durchführung von Updates der residenten Software gehört.

3. Verfahren nach Anspruch 1 oder 2, **dadurch gekennzeichnet, dass** ein externes Datenspeichergerät (40) vorgesehen ist, das an das Prozessorsystem (10) anschließbar ist, und die nicht-residente Software in das Prozessorsystem zu laden.

4. Verfahren nach Anspruch 3, **dadurch gekennzeichnet, dass** das Prozessorsystem (10) imstande ist, Angaben über seine gespeicherte Software und Daten an das externe Datenspeichergerät (40) zu übertragen.

5. Verfahren nach Anspruch 3 oder 4, **dadurch gekennzeichnet, dass** zu der residenten Software (20) ein Programm gehört, das ein spezifisches Programm für das angeschlossene externe Datenspeichergerät (40) aus dem externen Datenspeichergerät (40) lädt, als nicht-residente Software speichert und diese startet.

6. Verfahren nach einem der Ansprüche 3 bis 5, **dadurch gekennzeichnet, dass** das externe Datenspeichergerät (40) zum Speichern von Behandlungsinformationen und/oder Serviceinformationen aus dem Prozessorsystem (10) benutzt wird.

7. Verfahren nach einem der Ansprüche 3 bis 6, **dadurch gekennzeichnet, dass** das externe Datenspeichergerät (40) für ein Update der residenten Software (20, 22) benutzt wird.

8. Verfahren nach einem der Ansprüche 3 bis 7, **dadurch gekennzeichnet, dass** die Ein-/Ausgabe des externen Datenspeichergerätes (40) durch eine residente Ausführungssoftware (22) des Prozessorsystems (10) gesteuert wird.

9. Verfahren nach einem der Ansprüche 3 bis 7, **dadurch gekennzeichnet, dass** die Ein-/Ausgabe des externen Datenspeichergerätes (40) durch eine nicht-residente Ausführungssoftware (26) des Prozessorsystems (10) gesteuert wird.

10. Verfahren nach einem der Ansprüche 3 bis 9, **dadurch gekennzeichnet, dass** die Ein-/Ausgabe des externen Datenspeichergerätes (40) durch eine im externen Datenspeichergerät gespeicherte Ausführungssoftware von einem eigenen Prozessor des externen Datenspeichergerätes (40) gesteuert wird.

11. Patientenbehandlungsmaschine mit mindestens einem Prozessorsystem (10), das eine CPU (12) sowie Speichereinrichtungen und darin gespeicherte Software enthält, **dadurch gekennzeichnet, dass** ein Teil der Software, nämlich eine die Patientenbehandlung steuernde und überwachende Behandlungssoftware, in einem nicht-flüchtigen Speicher (14) gespeichert ist, während eine die Serviceaktivitäten und die Produktionsaktivitäten steuernde und überwachende Tool-Software in einem flüchtigen Speicher (24) gespeichert ist.

12. Patientenbehandlungsmaschine nach Anspruch 11, **dadurch gekennzeichnet, dass** ein an das Prozessorsystem (10) anschließbares externes Datenspeichergerät (40) vorgesehen ist, das die Behandlungssoftware und die Tool-Software enthält.

13. Patientenbehandlungsmaschine nach Anspruch 12, **dadurch gekennzeichnet, dass** das xterne Datenspeichergerät (40) ein löschbarer Speicher, insbesondere ein Speicherstick, ist.

14. Patientenbehandlungsmaschine nach Anspruch 12, **dadurch gekennzeichnet, dass** das externe Datenspeichergerät (40) einen eigenen Prozessor enthält.
